# FASCICULE DE BREVET EUROPEEN

(11) **EP 2 771 318 B1**
(45) Date de publication et mention de la délivrance du brevet: **06.06.2018**
(21) Numéro de dépôt: 12780246.0
(22) Date de dépôt: 12.10.2012
(51) Int. Cl.: C07D 207/16, A61K 8/42, A61K 8/49, C07C 233/47, C07C 233/49, A61P 17/00, A61K 31/401, A61Q 19/00

(54) **NOUVEAUX ESTERS DE DERIVES N-ACYLES D'ACIDES AMINES ET DE DIOLS, PROCEDE POUR LEUR PREPARATION, ET UTILISATION EN COSMETIQUE ET COMME MEDICAMENT**
NEUE ESTER AUS N-ACYLIERTEN AMINOSÄURE- UND DIOLDERIVATEN, VERFAHREN ZU IHRER HERSTELLUNG SOWIE IHRE VERWENDUNG IN DER KOSMETIK UND ALS ARZNEIMITTEL
NOVEL ESTERS OF N-ACYL DERIVATIVES OF AMINO ACIDS AND DIOLS, METHOD FOR PREPARING SAME, AND USE THEREOF IN COSMETICS AND AS A DRUG

(30) Priorité: 27.10.2011 FR 1159762
(43) Date de publication de la demande: 03.09.2014
(73) Titulaire: Société d'Exploitation de Produits pour les Industries Chimiques SEPPIC, 75007 Paris (FR)
(72) Inventeur: DUMONT, Sandy, F-81200 Caucalieres (FR); GUILBOT, Jérôme, F-81100 Castres (FR); GARCEL, Stéphanie, F-81100 Castres (FR); CATTUZZATO, Laetitia, F-81100 Castres (FR)
(74) Mandataire: Laigneau, Amandine
(86) Numéro de dépôt international: PCT/FR2012/052324
(87) Numéro de publication internationale: WO 2013/060964

(56) Documents cités:
- EP-A2- 0 839 515
- EP-A2- 1 216 696
- JP-A- 2012 031 216

## Description

La présente invention concerne de nouveaux produits chimiques, et de nouvelles compositions chimiques, destinés à la prévention et/ou au traitement des signes visibles du dysfonctionnement du système veineux et/ou de l'altération de la perméabilité vasculaire de la peau humaine.

La peau humaine constitue la première image offerte au regard d'autrui, et par conséquent, l'amélioration de son aspect est un sujet de préoccupation constant pour les êtres humains. La peau est le reflet d'un état de bien-être, souvent associé à la jeunesse, et a *contrario* d'un état de fatigue et/ou de vieillissement.

Un bon fonctionnement de la microcirculation sanguine constitue un des facteurs essentiels qui régissent le bon état esthétique cutané. Le phénomène de vieillissement se traduit notamment par une diminution du nombre, de la taille et de la fonctionnalité des vaisseaux dermiques qui assurent le fonctionnement de la microcirculation sanguine, ont tendance à devenir moins nombreux et plus fragiles.

Cette raréfaction des vaisseaux capillaires sous-cutanés entraine une moindre oxygénation de la peau et une diminution de l'apport en nutriments (oligo-éléments et vitamines), ce qui se traduit chez l'être humain âgé par une pâleur faciale chronique (références bibliographiques (1), (2) et (3). De même, tout dysfonctionnement du système veineux, qui se caractérise par un ralentissement de la circulation sanguine, produit le même phénomène d'oxygénation insuffisante du tissu connu sous le nom d'hypoxie.

La notion de perméabilité vasculaire décrit le rôle joué par de petits vaisseaux sanguins (artérioles, veinules ou micro-vaisseaux) pour apporter une fonction de barrière entre le sang circulant dans lesdits petits vaisseaux sanguins et les tissus, plus particulièrement les tissus cutanés. Lorsque la perméabilité vasculaire est altérée sous l'effet du vieillissement et/ou de phénomènes inflammatoires et/ou de stress d'origine extérieure, les tissus alors en situation d'hypoxie sont enflammés, ce qui se manifeste par l'apparition de phénomènes de rougeurs exacerbés, pouvant aller jusqu'à la formation d'oedèmes sur la zone cutanée concernée. Les vaisseaux peuvent par ailleurs se dilater, voire se rompre, entraînant la formation de télangiectasies.

L'endothélium est un tissu dont la fonction première est de contenir le sang à l'intérieur des vaisseaux sanguins, en permettant l'échange de substances nutritives avec le milieu intérieur. Il est constitué par les cellules endothéliales et par les cellules musculaires, qui agissent comme des « filtres » moléculaires pour permettre cet échange de substances nutritives, dont la fonction est de contrôler la coagulation sanguine et la vasomotricité de l'individu. Les médiateurs humoraux, les hormones, les cytokines ou les facteurs de croissance, constituent des contraintes biochimiques qui agissent sur l'activation des cellules endothéliales. Les cellules endothéliales sont sensibles au stress oxydatif, provoqué par une présence accrue de dérivés oxygénés, comme par exemple des ions superoxydes, des peroxydes d'hydrogène, des radicaux hydroxyles, qui dépassent les capacités régulatrices du système antioxydant naturel (superoxydismutase, catalase...), ce qui se traduit notamment par une diminution d'oxygène disponible dans les cellules endothéliales à savoir d'un phénomène d'hypoxie desdites cellules endothéliales, et par conséquent par une baisse de la production d'Adénine TriPhosphate (ATP) dans lesdites cellules endothéliales.

Selon la littérature (référence bibliographique (4) Janssens, « effect of venotropic drugs on the respiratory activity of isolated mitochondria and in endothelial cells », in British Journal of Pharmacology (2000) 130, 1513-1524*),* les insuffisances veineuses, résultant de dysfonctionnement du système veineux et/ou de l'altération de la perméabilité vasculaire, qui se traduisent par la diminution de l'apport sanguin artériel à un organe (ou ischémie), laquelle entraine essentiellement une baisse de l'oxygénation des tissus de l'organe en dessous de ses besoins pour le placer en situation d'hypoxie et par conséquent une diminution de la production d'ATP par les cellules endothéliales.

Selon l'organisation des cellules endothéliales dans les organes, l'endothélium remplit une fonction spécifique dudit organe. De ce fait, lorsqu'elles sont soumises à une contrainte mécanique ou biochimique, les cellules endothéliales génèrent des réponses aux stimuli de natures différentes (exposition aux rayonnements Ultra-Violets, variation importante de température et/ou d'hygrométrie, pollution, ...), qui ont des conséquences macroscopiques différentes. Ainsi, un dysfonctionnement du système veineux et/ou de l'altération de la perméabilité vasculaire, généré et/ou exacerbé par des contraintes mécaniques et/ou biochimiques, peut se traduire par une situation d'hypoxie dans la région du contour de l'oeil se manifestant par l'apparition d'oedème de caractère non inflammatoire, et notamment par l'apparition de cernes et/ou de poches sous les yeux, ou au niveau des membres inférieurs, par l'apparition de sensations de lourdeur des jambes se traduisant notamment par un gonflement du mollet et/ou des pieds et/ou des chevilles.

La région du contour de l'oeil se caractérise par une innervation dense et par une peau fine, peu riche en lipides cutanés, se montrant alors très sensible aux stress extérieurs (état de fatigue, manque de sommeil, exposition aux UV, tabac, alcool,...) et aux différentes contraintes mécaniques et biochimiques. Les dysfonctionnements du système veineux et/ou l'altération de la perméabilité vasculaire qui se traduisent par une vasodilatation ou une congestion des capillaires sanguins dans cette zone particulière du contour de l'oeil sont également plus visible du fait de la minceur de la peau. Lorsque la vasodilatation ou la congestion des capillaires sanguins présents sous les yeux perdurent, ces phénomènes engendrent des sensations d'inconfort persistantes et provoquent l'apparition de cernes et/ou de poches sous les yeux qui revêtent alors un caractère inesthétique. Le vieillissement cutané se traduit également par une diminution du nombre, de la taille et de la fonctionnalité des vaisseaux dermiques, ce qui entraîne une diminution de l'apport nutritionnel et de l'éclat du teint. Ces phénomènes sont également confortés par une circulation lymphatique ralentie dans cette zone du contour de l'oeil.

En ce qui concerne le phénomène ou la sensation de lourdeur au niveau des membres inférieurs, et notamment le phénomène dit de «jambes lourdes », il est ressenti par les sujets qui présentent des dysfonctionnements du système veineux et/ou une altération de la perméabilité vasculaire, déclenchés ou aggravés par des facteurs liés à l'hérédité, à la sédentarité, à une station debout prolongée, à l'exposition à la chaleur, à l'abus de tabac ou d'alcool. Ce phénomène se caractérise par une dilatation des veines, et se manifeste par l'apparition de douleurs, de fourmillements et de gonflement du mollet, des pieds et des chevilles.

Il existe donc un besoin de disposer de solutions satisfaisantes pour prévenir et/ou traiter les diminutions de productions de l'ATP par les cellules endothéliales sous l'effet de stress oxydants, de façon à prévenir et/ou traiter les dysfonctionnements du système veineux et/ou l'altération de la perméabilité vasculaire qui se traduisent par l'hypoxie des cellules endothéliales du corps humain et par des effets inesthétiques, comme par exemple les cernes et/ou les poches péri-oculaires et le phénomène de « jambes lourdes ».

Les produits de maquillage constituent une solution qui permet de masquer, d'atténuer les défauts apparents de la peau, et peuvent présenter une solution à la présence de cernes et de poches dans la zone péri-oculaire. Les fonds de teint procurent un aspect mat à la peau et permettent d'unifier son teint. Mais ces solutions cosmétiques ne permettent de traiter que les conséquences apparentes des dysfonctionnements du système veineux ou de l'altération de la perméabilité vasculaire sur la seule zone péri-oculaire sans traiter leurs causes. De plus, l'utilisation de ces compositions de maquillage présentent l'inconvénient de conférer à la peau un aspect non naturel et certaines d'entre elles sont difficiles à étaler et peuvent provoquer un assèchement de la peau sur le long terme.

Une autre solution consiste à favoriser la production de monoxyde d'azote par les mitochondries des cellules sujettes au phénomène d'hypoxie. Le monoxyde d'azote est une molécule connue qui est libérée notamment par les cellules endothéliales ce qui permet de provoquer le phénomène de vasodilatation et par conséquent un accroissement du débit sanguin. La publication internationale WO2008/141296A1 décrit un procédé de traitement de l'hypoxie de tissus de mammifères en exposant lesdits tissus à des rayonnements électromagnétiques dans la partie visible du spectre de la lumière, de façon à favoriser la production de monoxyde d'azote par les mitochondries des tissus exposés à ces rayonnements. La publication FR 2 883 171 A1 décrit l'utilisation d'agents favorisant la production de monoxyde d'azote dans et/ou sur la peau, choisis parmi des donneurs ou des précurseurs de monoxyde d'azote (comme par exemple les composés comprenant des substituants nitro ou nitroso, des oximes, l'hydroxylamine, la N-hydroxy guanidine et ses sels, des métaux de transition nitrosylés, ...), des agents permettant la libération non polymérique de monoxyde d'azote dans l'organisme (comme par exemple des acides aminés, des peptides), des agents permettant la stimulation de la synthèse et/ou de l'activité monoxyde d'azote synthase (NOS) comme par exemple des interleukines, des lipopolysaccharides, l'acide L-glutamique, l'acide arachidonique. Cette approche par la mise en oeuvre de solutions visant à générer une augmentation de la production de monoxyde d'azote dans l'organisme présente cependant comme inconvénients de ne concerner que la stimulation du phénomène de vasodilatation et d'induire des risques de dérégulation de la balance vasodilatation / vasoconstriction ; l'alternance et l'équilibre des deux phénomènes étant à respecter pour conserver et/ou retrouver un fonctionnement du système veineux et/ou une perméabilité vasculaire équilibrés.

Une autre solution consiste à favoriser la chélation des ions Fe³⁺, présents dans l'hémosidérine qui est un pigment résultant de la dégradation de l'hémoglobine accumulée dans les vaisseaux capillaires du fait d'un ralentissement de la microcirculation sanguine dans la région péri-oculaire. La publication internationale WO2008035152 A1 décrit plusieurs agents chélateurs des ions ferriques efficaces et ne présentant pas de problèmes d'irritation oculaire lors de l'application d'une formulation les contenant sur la zone à préserver ou à traiter.

Parmi ces agents, on peut citer : la 3-hydroxy 2-méthy 4-pyrone (ou maltol), l'éthyl maltol, l'octopirox, le ciclopirox, le rilopirox, l'acide gallique, les esters de l'acide gallique, l'acide kojique, les dérivés de l'acide kojique. Cette solution, outre qu'elle met en oeuvre des composés soit en mélange dans des extraits végétaux soit obtenus à l'issue de procédés multi-étapes non adaptés à l'industrie cosmétique, ne permet de traiter que les conséquences apparentes des dysfonctionnements du système veineux ou de l'altération de la perméabilité vasculaire sur la seule zone péri-oculaire sans traiter leurs causes.

La demande de brevet japonais No 2000-229921 décrit l'utilisation de polyols esters de N-acylamino acides comme agent tensioactifs performants (paragraphe [0003] de ladite demande). La publication internationale WO2010034917 décrit les mono-esters et les diesters de polyols du N-(ω-undécylenoyl) phenylalanine et leurs utilisations comme agents éclaircissants de la peau humaine.

La demande de brevet européen No EP 0 839 515 A2 divulgue de façon générique des esters de N-acylamino acides, et leur utilisation comme agent de promotion de croissance des cheveux, comme agent humectant et comme agent permettant l'accélération du flux sanguin sous-cutané. La demande de brevet européen No EP 0 839 515 A2 divulgue plus particulièrement de façon expresse un ester d'éthylène glycol du N-pentadécanoyl aspartate, ainsi que son utilisation comme agent de promotion de la croissance des cheveux.

A notre connaissance, aucun ester de dérivés N-acyl aminoacides et de diols aliphatiques n'a été décrit comme capable de prévenir et/ou de traiter les diminutions de productions de l'ATP par les cellules endothéliales soumises à des stress oxydants. Par conséquent, à notre connaissance, aucun ester de dérivés N-acyl aminoacides et de diols aliphatiques n'a été décrit comme capable de prévenir et/ou de traiter les dysfonctionnements du système veineux et/ou de l'altération de la perméabilité vasculaire.

De même, à notre connaissance, aucun ester de dérivés N-acyl aminoacides et de diols aliphatiques n'a été décrit comme capable de prévenir l'apparition et/ou de diminuer les effets inesthétiques générés par l'hypoxie des cellules endothéliales du corps humain, comme par exemple les cernes et/ou les poches péri-oculaires et le phénomène de « jambes lourdes ».

La demanderesse s'est donc attachée à développer une solution technique nouvelle, consistant en de nouveaux esters de dérivés N-acyl aminoacides et de diols aliphatiques, qui permettent de prévenir et/ou de ralentir la baisse de la production de l'ATP par les cellules endothéliales soumises à des stress oxydants, de façon à prévenir et/ou à traiter les dysfonctionnements du système veineux et/ou de l'altération de la perméabilité vasculaire, et par conséquent de prévenir l'apparition et/ou de diminuer les effets inesthétiques générés par l'hypoxie des cellules endothéliales du corps humain, comme par exemple les cernes et/ou les poches péri-oculaires et le phénomène de « jambes lourdes ».

C'est pourquoi, selon un premier aspect, l'invention a pour objet la composition (C1) comprenant pour 100% de sa masse :
- De 99% massique à 20% massique d'au moins un composé de formule (la)

   R'-O-A-O-H (la),

   formule (la) dans laquelle R' représente soit un radical monovalent de formule (IIa) : 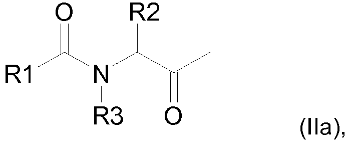 dans laquelle R1 représente un radical aliphatique, saturé ou insaturé, linéaire ou ramifié, comportant de 7 à 30 atomes de carbone, R2 représente un atome d'hydrogène ou un radical choisi parmi les radicaux méthyle, isopropyle, isobutyle, 1-méthyl propyle, hydroxyméthyle, 1-hydroxy éthyle, thiométhyle, 2-méthylthio éthyle, 4-aminobutyle, 3-guanidino propyle, 3-uréido propyle, (1-amino carbonyl) méthyle, 2-(amino carbonyl) éthyle, benzyle, 4-hydroxy benzyle, 3,4-dihydroxy benzyle, [1H-indol-3-yl] méthyle, (1H-imidazol-4-yl) méthyle, 3-amino propyle et R3 représente un atome d'hydrogène ou un radical méthyle ; soit un radical monovalent de formule (IIb) : 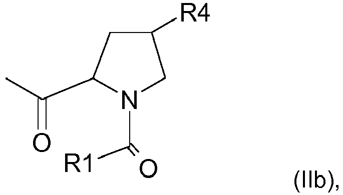 dans laquelle R1 est tel que défini dans la formule (IIa) et R4 représente un atome d'hydrogène ou un radical hydroxy ;
   et formule (la) dans laquelle A représente le radical divalent de formule (III) :

   -CH(X1)-[C(X2)(X3)]p-CH(X4)- (III)

   dans laquelle X1, X2, X3 et X4, identiques ou différents, représentent soit un atome d'hydrogène, soit un radical méthyle, soit un radical éthyle et p représente un nombre entier supérieur ou égal à 1 et inférieur ou égal à 6 ; et
- De 1% massique à 80% massique d'au moins un composé de formule (Ib) :

   R-O-A-O-R (Ib),

   formule (Ib) dans laquelle R, représente soit un radical monovalent de formule (IIa) : 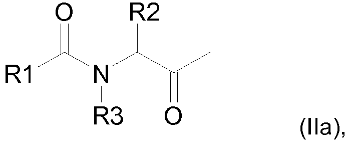 dans laquelle R1 représente un radical aliphatique, saturé ou insaturé, linéaire ou ramifié, comportant de 7 à 30 atomes de carbone, R2 représente un atome d'hydrogène ou un radical choisi parmi les radicaux méthyle, isopropyle, isobutyle, 1-méthyl propyle, hydroxyméthyle, 1-hydroxy éthyle, thiométhyle, 2-méthylthio éthyle, 4-aminobutyle, 3-guanidino propyle, 3-uréido propyle, (1-amino carbonyl) méthyle, 2-(amino carbonyl) éthyle, benzyle, 4-hydroxy benzyle, 3,4-dihydroxy benzyle, [1H-indol-3-yl] méthyle, (1H-imidazol-4-yl) méthyle, 3-amino propyle, et R3 représente un atome d'hydrogène ou un radical méthyle ; soit un radical monovalent de formule (IIb) : 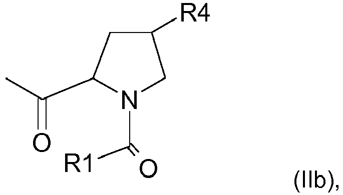 dans laquelle R1 est tel que défini dans la formule (IIa) et R4 représente un atome d'hydrogène ou un radical hydroxy,
   et formule (Ib) dans laquelle A représente le radical divalent de formule (III) :

   -CH(X1)-[C(X2)(X3)]p-CH(X4)- (III)

   dans laquelle X1, X2, X3 et X4, identiques ou différents, représentent soit un atome d'hydrogène, soit un radical méthyle, soit un radical éthyle et P représente un nombre entier supérieur ou égal à 1 et inférieur ou égal à 6.

Selon un aspect particulier, dans la définition du radical de formule (IIa) ou du radical de formule (IIb), le radical RᵣC(=O)- représente un radical choisi parmi les radicaux octanoyle, décanoyle, ω-undécylènoyle, dodécanoyle, tétradécanoyle, hexadécanoyle, octadécanoyle, eicosanoyle, docosanoyle, 9-octadécènoyle, éicosènoyle, 13-docosènoyle, 9,12-octadécadiènoyle ou 9,12,15-octadécatriénoyle. radicaux octanoyle, décanoyle, ω-undécylènoyle, dodécanoyle, tétradécanoyle, hexadécanoyle, octadécanoyle, eicosanoyle, docosanoyle, 9-octadécènoyle, éicosènoyle, 13-docosènoyle, 9,12-octadécadiènoyle ou 9,12,15-octadécatriénoyle.

Selon un autre aspect particulier de la présente invention, celle-ci a pour objet une composition (C1) telle que définie précédemment, pour laquelle dans la formule (IIa), R1 et R3 sont tels que définis précédemment et R2 représente un radical choisi parmi les radicaux, méthyle, isopropyle, isobutyle, 1-méthyl propyle ou benzyle, et dans la formule (IIb), R1 est tel que défini précédemment et R4 représente un atome d'hydrogène.

Selon cet aspect particulier de la présente invention, les composés de formule (Ia) et (Ib) telles que définies ci-dessus sont plus particulièrement choisi parmi les esters dérivés des acides aminés suivants : l'alanine, la valine, la proline, la leucine, la phénylalanine, l'isoleucine.

Selon un autre aspect particulier de la présente invention, celle-ci a pour objet une composition (C1) telle que définie précédemment, pour laquelle, dans les formules (la) et (Ib), A représente le radical divalent de formule (IIIa) :

-(CH₂)_{q}- (IIIa)

Dans laquelle q est égal 3, 4 ou à 6, correspondant à la formule (III) dans laquelle X1, X2, X3, et X4 sont identiques et représentent chacun un atome d'hydrogène, et p représente un nombre entier égal à 1, 2 ou 4.

Selon cet aspect particulier de la présente invention, les composés de formule (la) et (Ib) telles que définies ci-dessus sont alors choisi parmi les esters du 1,3-propanediol lorsque p représente un nombre entier égal à 1, les esters du 1,4- butanediol lorsque p représente un nombre entier égal à 2, les esters du 1,6-hexanediol lorsque p représente un nombre entier égal à 4.

Selon cet aspect particulier de la présente invention, dans les composés de formule (la) et (Ib), telle que définie ci-dessus A représente plus particulièrement le radical divalent - (CH₂)₃-.

Selon un autre aspect particulier de la présente invention, celle-ci a pour objet une composition (C1) telle que définie précédemment, pour laquelle, dans les formules (la) et (Ib), X1, X2 et X3 sont identiques et représentent un atome d'hydrogène, X4 représente un radical aliphatique sélectionné parmi les radicaux méthyle, éthyle, propyle, butyle, pentyle, hexyle, heptyle, octyle, nonyle et décyle, et p représente un nombre entier égal à 1 dans la définition de la formule (III) du radical divalent A.

Selon cet aspect particulier de la présente invention, les composés de formules (Ia) et (Ib) sont caractérisés en ce que A représente le radical divalent -(CH₂)₂-CH(CH₃)-.

Selon un autre aspect particulier, dans la composition (C1), le composé de formule (la) est l'un des composés suivants :
- Le composé de formule (Ia11) : 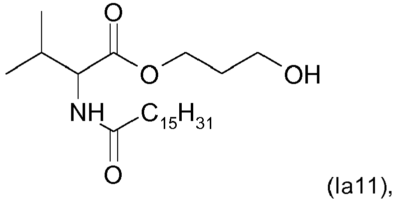
- Le composé de formule (Ia12) : 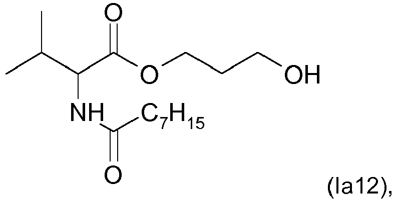
- Le composé de formule (Ia13) : 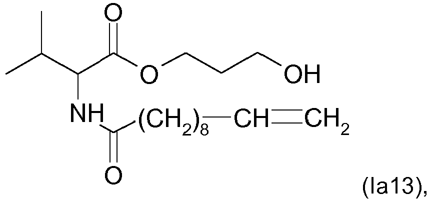
- Le composé de formule (Ia14) : 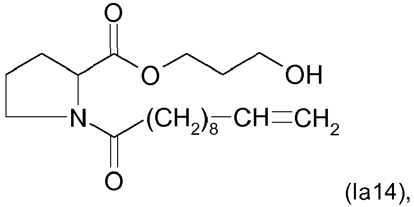
- Le composé de formule (Ia15) : 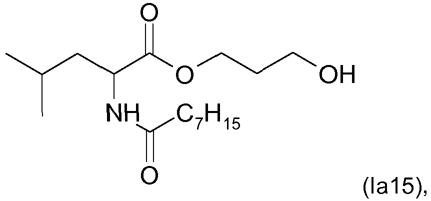
- Le composé de formule (Ia21) : 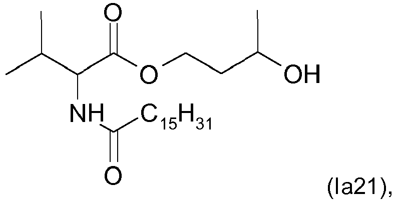
- Le composé de formule (Ia22) : 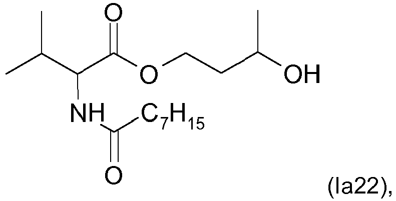
- Le composé de formule (Ia23) : 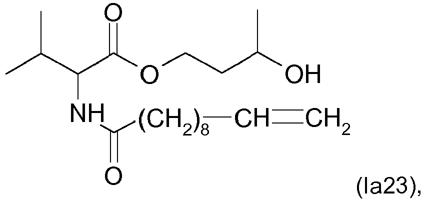
- Le composé de formule (Ia24) : 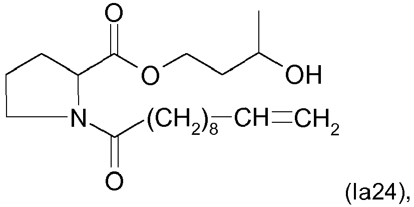
- Le composé de formule (Ia25) : 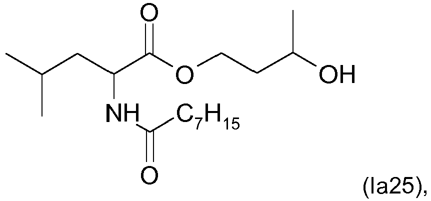
- Le composé de formule Ia31) : 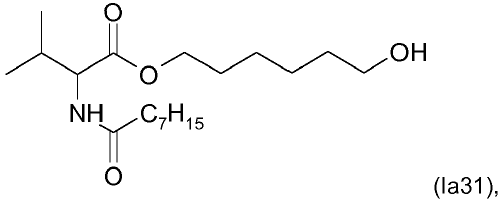
- Le composé de formule (Ia32) : 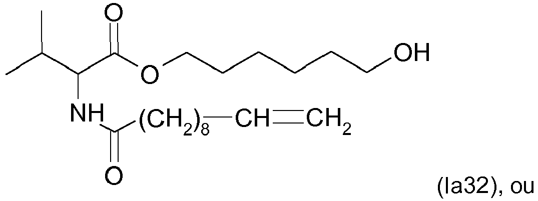
- Le composé de formule (Ia33) : 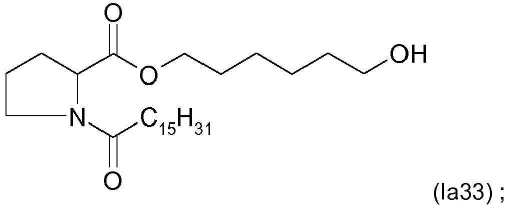
et plus particulièrement l'un des composés suivants :
- Le mono N-hexadécanoyl valinate de 1,3 propanediol,
- Le mono N-octanoyl valinate de 1,3 propanediol,
- Le mono N-(ω-undecylènoyl) valinate de 1,3 propanediol,
- Le mono N-(ω-undecylènoyl) prolinate de 1,3 propanediol,
- Le mono N-octanoyl leucinate de 1,3 propanediol,
- Le mono N-hexadécanoyl valinate de 1,3 butanediol,
- Le mono N-octanoyl valinate de 1,3 butanediol,,
- Le mono N-(ω-undecylènoyl) valinate de 1,3 butanediol,
- Le mono N-(ω-undecylènoyl) prolinate de 1,3 butanediol,
- Le mono N-octanoyl leucinate de 1,3 butanediol,
- Le mono N-octanoyl valinate de 1,6 hexanediol,
- Le mono N-(ω-undecylènoyl) valinate de 1,6 hexanediol, ou
- Le mono N-hexadécanoyl prolinate de 1,6 hexanediol ;
tandis que le composé de formule (Ib) est l'un des composés suivants :
- Le composé de formule (Ib11) : 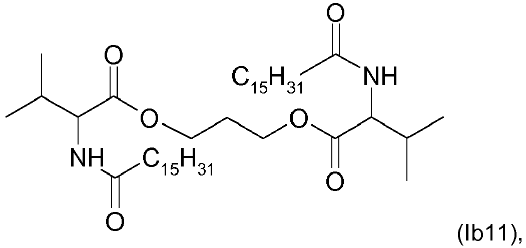
- Le composé de formule (Ib12) : 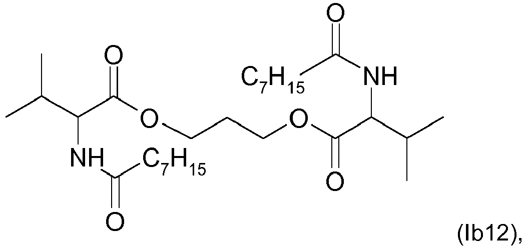
- Le composé de formule (Ib13) : 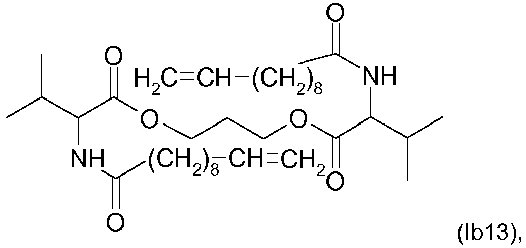
- Le composé de formule (Ib14) : 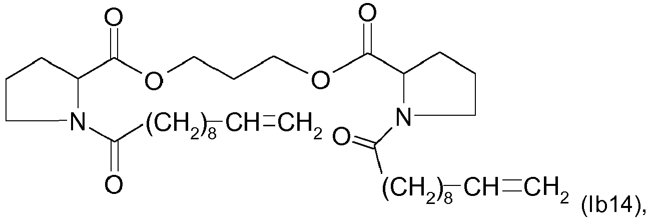
- Le composé de formule (Ib15) : 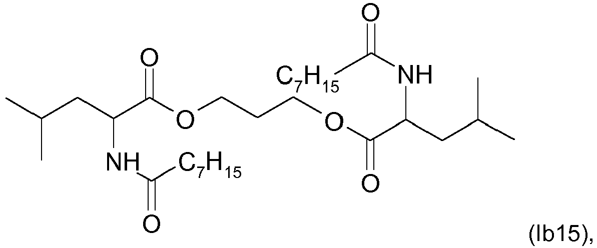
- Le composé de formule (Ib21) : 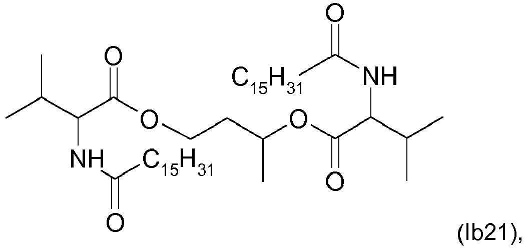
- Le composé de formule (Ib22) : 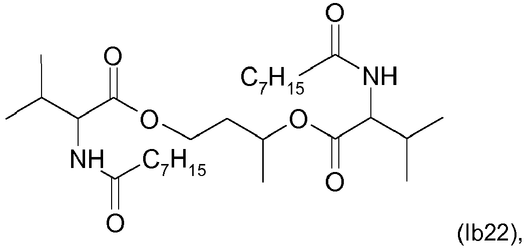
- Le composé de formule (Ib23) : 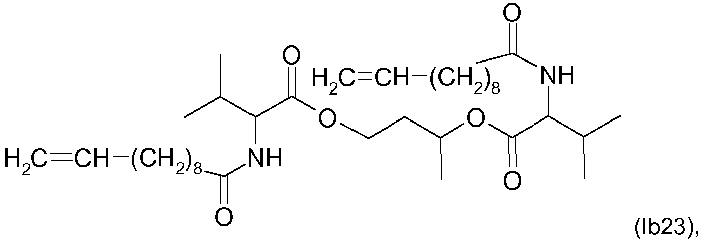
- Le composé de formule (Ib24) : 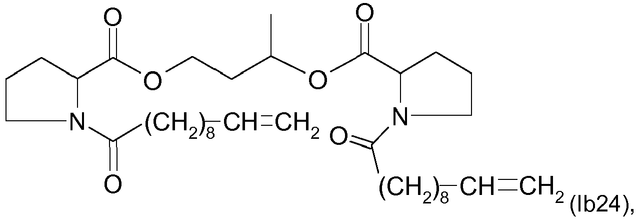
- Le composé de formule (Ib25) : 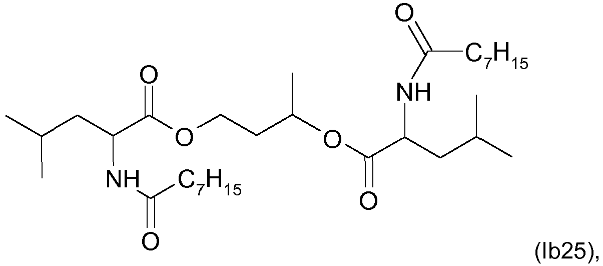
- Le composé de formule (Ib31) : 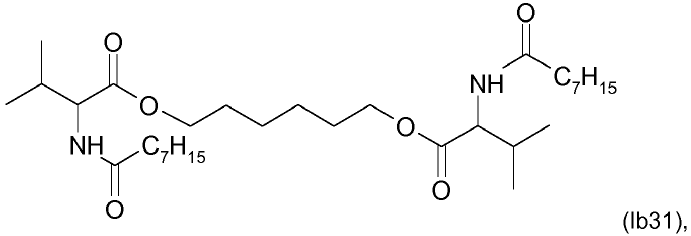
- Le composé de formule (Ib32) : 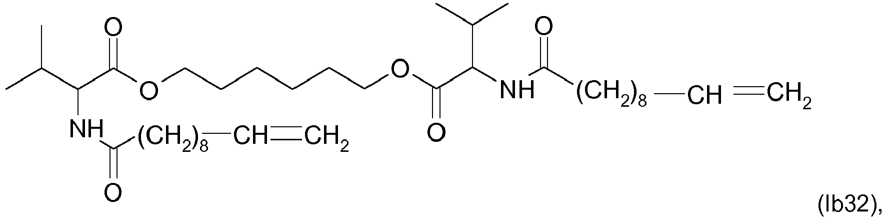 ou
- Le composé de formule (Ib33) : 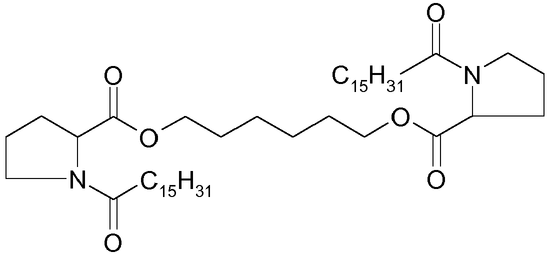
et plus particulièrement l'un des composés suivants :
- Le di N-hexadécanoyl valinate de 1,3 propanediol,
- Le di N-octanoyl valinate de 1,3 propanediol,
- Le di N-(ω-undecylènoyl) valinate de 1,3 propanediol,
- Le di N-(ω-undecylènoyl) prolinate de 1,3 propanediol,
- Le di N-octanoyl leucinate de 1,3 propanediol,
- Le di N-hexadécanoyl valinate de 1,3 butanediol,
- Le di N-octanoyl valinate de 1,3 butanediol,,
- Le di N-(ω-undecylènoyl) valinate de 1,3 butanediol,
- Le di N-(ω-undecylènoyl) prolinate de 1,3 butanediol,
- Le di N-octanoyl leucinate de 1,3 butanediol,
- Le di N-octanoyl valinate de 1,6 hexanediol,
- Le di N-(ω-undecylènoyl) valinate de 1,6 hexanediol, ou
- Le di N-hexadécanoyl prolinate de 1,6 hexanediol.

La composition (C₁) objet de l'invention peut être préparée par diverses voies.

Une première voie de préparation de la composition (C₁) objet de l'invention consiste à mélanger dans les proportions massiques souhaitées, le composé de formule (Ia) tel que définie ci-dessus ou le mélange de composés de formule (Ia), avec le composé de formule (Ib) telle que définie ci-dessus, ou le mélange de composés de formule (Ib).

Une seconde voie de préparation de la composition (C₁) objet de l'invention consiste à mettre en oeuvre le procédé de préparation du composé de formule (I) tel que décrit précédemment, en faisant réagir dans les proportions souhaitées, le composé de formule (V) avec le composé de formule (IVa) ou de formule (IVb) ou un mélange de composés de formule (IVa) et de formule (IVb).
Une troisième voie de préparation de la composition (C1) objet de l'invention consiste à à faire réagir dans les proportions souhaitées, le composé de formule (V) :

H-O-A-O-H (V),

Dans laquelle A représente un radical divalent de formule (III) telle que définie précédemment, avec le composé de formule (VIIa) : 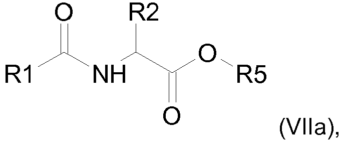 Dans laquelle R1, R2 et R5 sont tels que définis précédemment, ou le composé de formule (VIIb) : 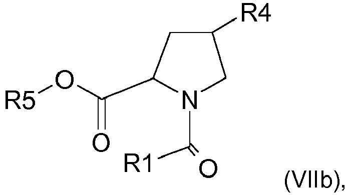 Dans laquelle R1, R4 et R5 sont tels que définis précédemment.

L'invention a aussi pour objet l'utilisation de la composition (C1) telles que définie précédemment, comme agent actif cosmétique, pour prévenir et/ou limiter les effets inesthétiques générés par l'hypoxie des cellules endothéliales du corps humain et plus particulièrement ceux générés par les cernes, les poches péri-oculaires et/ou les jambes lourdes.

La composition (C1) objet de la présente invention peut être administrée par voie orale, topique ou parentérale.

L'invention a aussi pour objet une formulation cosmétique à usage topique caractérisée en ce qu'elle comprend au moins un excipient cosmétiquement acceptable et une quantité efficace de la composition (C1) telle que définie précédemment.

L'expression "à usage topique" utilisée dans la définition de la formulation cosmétique telle que décrite ci-dessus, signifie que ladite formulation est mise en oeuvre par application sur la peau, qu'il s'agisse d'une application directe dans le cas d'une formulation cosmétique ou d'une application indirecte par exemple dans le cas d'un produit de soin corporel sous forme de lingette en textile ou en papier ou de produits sanitaires destinés à être en contact. avec la peau.

L'expression « cosmétiquement acceptable » utilisée dans la définition de la formulation cosmétique telle que décrite ci-dessus, signifie selon la directive du Conseil de la Communauté Economique Européenne N°76/768/CEE du 27 juillet 1976 modifiée par la directive N°93/35/CEE du 14 juin 1993, que ladite formulation comprend toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état.

L'invention a aussi pour objet un procédé de traitement de la peau humaine destiné à prévenir l'apparition et/ou à diminuer les cernes et/ou les poches péri-oculaires et/ou le phénomène de jambes lourdes comprenant au moins une étape d'application sur ladite peau humaine d'une quantité efficace de la formulation cosmétique à usage topique telle que définie ci-dessus.

Par quantité efficace d'une composition (C1) telle que définie précédemment, présents dans la formulation cosmétique à usage topique telle que définie précédemment, destinés à prévenir et/ou à limiter les effets inesthétiques générés par l'hypoxie des cellules endothéliales du corps humain et plus particulièrement ceux générés par les cernes, les poches péri-oculaires et/ou les jambes lourd, on entend pour 100% de la masse de ladite formulation cosmétique à usage topique la quantité comprise entre 0,1% et 5% massique, plus particulièrement entre 0,1% et 3% massique, et encore plus particulièrement entre 0,5% et 2.% massique de composition (C1).

Dans le procédé de traitement tel que décrit ci-dessus, la formulation cosmétique à usage topique est étalée sur la surface de la peau à traiter, puis la peau est massée quelques instants.

Les formulation cosmétique à usage topique objet de la présente invention, se présentent généralement sous forme de solutions aqueuses ou hydro-alcooliques diluées, sous forme d'émulsions simples ou multiples, telles que les émulsions eau dans huile (E/H), huile dans eau (H/E) ou eau dans huile dans eau (E/H/E), dans lesquelles l'huile est de nature végétale ou minérale, ou sous forme de poudre. Elles peuvent aussi être dispersées ou imprégnées sur du textile ou sur des matériaux non tissés qu'il s'agisse de lingettes, de serviettes en papier ou de vêtements.

De façon générale, la composition (C1) est associé à de nombreux types d'adjuvants ou de principes actifs utilisés dans la formulation cosmétique telle que définie ci-dessus et objet de la présente invention, qu'il s'agisse, de corps gras, de solvants organiques, d'épaississants, de gélifiants, d'adoucissants, d'agents tensioactifs moussants et/ou détergents, d'agents surgraissants, de tensioactifs épaississants et/ou gélifiants, d'antioxydants, d'opacifiants, de stabilisants, de moussants, de parfums, de tensioactifs émulsionnants, d'agents hydrotropes, d'agents plastifiants, d'agents surgraissants, d'agents de texture, de pigments, de séquestrants, de chélateurs, de conservateurs, d'huiles essentielles, de matières colorantes, d'actifs hydrophiles ou lipophiles, d'humectants, de parfums, de filtres solaires minéraux ou organiques, de charges minérales, ou tout autre ingrédient habituellement utilisé en cosmétique.

Comme exemples d'huiles que l'on peut associerà la composition (C1) dans les formulations cosmétiques à usage topique objet de la présente invention, on peut citer les huiles minérales telles que l'huile de paraffine, l'huile de vaseline, les isoparaffines ou les huiles blanches minérales, les huiles d'origine animale, telles que le squalène ou le squalane, les huiles végétales, telles que l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula ; les huiles végétales éthoxylées ; les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les polyalphaoléfines, les polyoléfines comme le polyisobutène, les isoalcanes de synthèse comme l'isohexadecane, l'isododécane, les huiles perfluorées et les huiles de silicone. Parmi ces dernières, on peut plus particulièrement citer les diméthylpolysiloxanes, méthylphénylpolysiloxanes, les silicones modifiées par des aminés, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Comme autre matière grasse que l'on peut associerà la composition (C1) dans les formulations cosmétiques à usage topique objet de la présente invention, on peut citer les alcools gras ou les acides gras.

Comme exemple de cires que l'on peut associerà la composition (C1) dans les formulations cosmétiques à usage topique objet de la présente invention, on peut citer par exemple la cire d'abeille ; la cire de carnauba ; la cire de candelilla ; la cire d'ouricoury ; la cire du Japon ; la cire de fibre de liège ou de canne à sucre ; les cires de paraffines ; les cires de lignite ; les cires microcristallines ; la cire de lanoline ; l'ozokérite ; la cire de polyéthylène ; les huiles hydrogénées ; les cires de silicone ; les cires végétales ; les alcools gras et les acides gras solides à température ambiante ; les glycérides solides à température ambiante.

Comme exemple de polymères épaississants et/ou émulsionnant que l'on peut associerà la composition (C1) dans les formulations cosmétiques à usage topique objet de la présente invention, on peut citer par exemple les homopolymères ou copolymères de l'acide acrylique ou de dérivés de l'acide acrylique, les homopolymères ou copolymères de l'acrylamide, les homopolymères ou copolymères de dérivés de l'acrylamide, les homopolymères ou copolymères de l'acide acrylamidométhyl propanesulfonique, de monomère vinylique, de chlorure de triméthylaminoéthylacrylate, les hydrocolloïdes d'origine végétale ou biosynthétique, par exemple la gomme de xanthane, la gomme de karaya, les carraghénates, les alginates ; les silicates ; la cellulose et ses dérivés ; l'amidon et ses dérivés hydrophiles ; les polyuréthanes.

Parmi les polymères de type polyélectrolytes que l'on peut associerà la composition (C1) dans les formulations cosmétiques à usage topique objet de la présente invention, on peut citer par exemple les copolymères de l'acide acrylique et de l'acide-2-méthyl-[(1-oxo-2-propényl)amino] 1-propane sulfonique (AMPS), les copolymères de l'acrylamide et de l'acide-2-méthyl-[(1-oxo-2-propényl)amino] 1-propane sulfonique, les copolymères de l'acide-2-méthyl-[(1-oxo-2-propényl)amino] 1-propane sulfonique et de l'acrylate de (2-hydroxyéthyle), l'homopolymère de l'acide-2-méthyl-[(1-oxo-2-propényl)amino] 1-propanesulfonique, l'homopolymère de l'acide acrylique, les copolymères du chlorure d'acryloyl éthyl triméthyl ammonium et de l'acrylamide, les copolymères de l'AMPS et de la vinylpyrolidone, les copolymères de l'acide acrylique et d'acrylates d'alkyle dont la chaîne carbonée comprend entre dix et trente atomes de carbone, les copolymères de l'AMPS et d'acrylates d'alkyle dont la chaîne carbonée comprend entre dix et trente atomes de carbone. De tels polymères sont commercialisés respectivement sous les appellations SIMULGEL™ EG, SEPIGEL™ 305, SIMULGEL™ NS, SIMULGEL™ 800 et SIMULGEL™ A par la demanderesse.

Comme exemples d'émulsionnants que l'on peut associerà la composition (C1) dans les formulations cosmétiques à usage topique objet de la présente invention, on peut citer par exemple les acides gras, les acides gras éthoxylés, les esters d'acide gras et de sorbitol, les esters d'acides gras éthoxylés, les polysorbates, les esters de polyglycérol, les alcools gras éthoxylés, les esters de sucrose, les alkylpolyglycosides, les alcools gras sulfatés et phosphatés ou les mélanges d'alkylpolyglycosides et d'alcools gras décrits dans les demandes de brevet français 2 668 080, 2 734 496, 2 756 195, 2 762 317, 2 784 680, 2 784 904, 2 791 565, 2 790 977, 2 807 435 et 2 804 432.

Comme exemples de tensioactifs moussants et/ou détergents que l'on peut associerà la composition (C1) dans les formulations cosmétiques à usage topique objet de la présente invention, on peut citer : les tensioactifs anioniques, cationiques, amphotères ou non ioniques topiquement acceptables habituellement utilisés dans ce domaine d'activité.

Parmi les tensioactifs anioniques que l'on peut associerà la composition (C1) dans les formulations cosmétiques à usage topique objet de la présente invention, on citera particulièrement les sels de métaux alcalins, les sels de métaux alcalino-terreux, les sels d'ammonium, les sels d'amines, les sels d'aminoalcools des composés suivants : les alkyléthers sulfates, les alkylsulfates, les alkylamidoéthersulfates, les alkylarylpolyéthersulfates, les monoglycérides sulfates, les alpha-oléfinesulfonates, les paraffines sulfonates, les alkylphosphates, les alkylétherphosphates, les alkylsulfonates, les alkylamidesulfonates, les alkylarylsulfonates, les alkylcarboxylates, les alkylsulfosuccinates, les alkyléthersulfosuccinates, les alkylamidesulfosuccinates, les alkylsulfoacétates, les alkylsarcosinates, les acyliséthionates, les N-acyltaurates, les acyllactylates.

Parmi les tensioactifs anioniques que l'on peut associerà la composition (C1) dans les formulations cosmétiques à usage topique objet de la présente invention, on citera également les dérivés N-acylés d'acides aminés, de peptides, de protéines dont la chaîne acyle comporte de 8 à 16 atomes de carbone; les sels d'acides gras, les sels d'acides d'huile de coprah éventuellement hydrogénée.

Parmi les tensioactifs amphotères que l'on peut associerà la composition (C1) dans les formulations cosmétiques à usage topique objet de la présente invention, on citera particulièrement les alkylbétaines, les alkylamidobétaines, les sultaines, les alkylamidoalkylsulfobétaines, les dérivés d'imidazolines, les phosphobétaïnes, les amphopolyacétates et les amphopropionates.

Parmi les tensioactifs cationiques que l'on peut associerà la composition (C1) dans les formulations cosmétiques à usage topique objet de la présente invention, on citera particulièrement les dérivés d'ammoniums quaternaires.

Parmi les tensioactifs non ioniques que l'on peut associerà la composition (C1) dans les formulations cosmétiques à usage topique objet de la présente invention, on citera particulièrement les alkylpolyglycosides dont la chaîne alkyle comporte de 8 à 16 atomes de carbone, les dérivés d'huile de ricin, les polysorbates, les amides de coprah, les N-alkylamines, les oxydes d'amines.

Comme exemples d'agents de texture que l'on peut associerà la composition (C1) dans les formulations cosmétiques à usage topique objet de la présente invention, on peut citer par exemple des dérivés N-acylés d'acides aminés, comme par exemple la lauroyl lysine commercialisée sous l'appellation AMINOHOPE™LL par la société AJINOMOTO, l'octenyl starch succinate commercialisé sous l'appellation DRYFLO™ par la société NATIONAL STARCH, le myristyl polyglucoside commercialisé par SEPPIC sous l'appellation MONTANOV 14, les fibres de cellulose, les fibres de coton, les fibres de chitosane, le talc, la séricite, le mica.

Comme exemples d'agents opacifiants et/ou nacrants que l'on peut associerà la composition (C1) dans les formulations cosmétiques à usage topique objet de la présente invention, on peut citer par exemple le palmitate de sodium, le stéarate de sodium, l'hydroxystéarate de sodium, le palmitate de magnésium, le stéarate de magnésium, l'hydroxystéarate de magnésium, le monostéarate d'éthylène glycol, le distéarate d'éthylène glycol, le monostéarate de polyéthylène glycol, le distéarate de polyéthylène glycol, les alcools gras.

Comme exemples de tensioactifs épaississants et/ou gélifiants que l'on peut associerà la composition (C1) dans les formulations cosmétiques à usage topique objet de la présente invention, on peut citer :
- Les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120.
- Les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.
- Les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples de filtres solaires que l'on peut associerà la composition (C1) dans les formulations cosmétiques à usage topique objet de la présente invention, on peut citer tous ceux figurant dans la directive cosmétique 76/768/CEE modifiée annexe VII.

Comme exemples de principe actif que l'on peut associerà la composition (C1) dans les formulations cosmétiques à usage topique objet de la présente invention, on peut citer les composés ayant une action éclaircissante ou dépigmentante tels que par exemple l'arbutine, l'acide kojique, l'hydroquinone, l'acide ellagique, la vitamine C, le magnésium ascorbyl phosphate, les extraits de polyphénols, les dérivés de polyphénols glycosylés comme le Rosmarinyl glucoside, les extraits de raisin, les extraits de pin, les extraits de vin, les extraits d'olives, les extraits de mare, les protéines N-acylées, les peptides N-acylés, les acides aminés N-acylés, les hydrolysâts partiels de protéines N-acylés, les acides aminés, les peptides, les hydrolysâts totaux de protéines, les hydrolysâts partiels de protéines, les polyols (par exemple, la glycérine ou le butylène glycol), l'urée, l'acide pyrrolidonecarboxylique ou les dérivés de cet acide, l'acide glycyrrhétinique, l'alpha-bisabolol, les sucres ou les dérivés des sucres, les polysaccharides ou leurs dérivés, les hydroxyacides par exemple l'acide lactique, les vitamines, les dérivés de vitamines comme le Rétinol, la vitamine E et ses dérivés, les minéraux, les enzymes, les co-enzymes, comme, le Coenzyme Q10, les hormones ou "hormone like", les extraits de soja par exemple, la Raffermine™, les extraits de blé par exemple la Tensine™ ou la Gliadine™, les extraits végétaux, tels que les extraits riches en tanins, les extraits riches en isoflavones ou les extraits riches en terpènes, les extraits d'algues d'eau douce ou marines, les cires essentielles, les extraits bactériens, les minéraux, les lipides en général, les lipides tels que les céramides ou les phospholipides, les actifs ayant une action amincissante comme la caféine ou ses dérivés, comme l'extrait de quinoa commercialisé sous l'appellation ADIPOLESS™, comme l'extrait de pruche canadienne commercialisé sous l'appellation SERENIKS™ 207, comme la composition comprenant du Lauroyl Proline commercialisée sous l'appellation ADIPOSLIM™, les actifs ayant une activité antimicrobienne ou une action purifiante vis à vis des peaux grasses tels que le LIPACIDE™ PVB, les actifs ayant une propriété énergisante ou stimulante comme le SEPITONIC™ M3 ou le Physiogényl™ le panthénol et ses dérivés comme le SEPICAP™ MP, les actifs anti-âge comme le SEPILIFT™ DPHP, le LIPACIDE™ PVB, le SEPIVINOL™, le SEPIVITAL™, les actifs hydratants comme le SEPICALM™ S, le SEPICALM™ VG et le SEPILIFT™ DPHP, les actifs anti-âge " anti-photo vieillissement ", les actifs protecteurs de l'intégrité de la jonction dermo-épidermique, les actifs augmentant la synthèse des composants de la matrice extracellulaire, les actifs ayant une activité amincissante comme la caféine, la théophylline, l'AMPc, le thé vert, la sauge, le ginko biloba, le lierre, le marron d'inde, le bambou, le ruscus, le petit houx, la centella asiatica, la bruyère, l'ulmaine, le fucus, le romarin, la saule, des actifs créant une sensation de « chauffe » sur la peau comme les activateurs de la microcirculation cutanée (exemple des nicotinates) ou des produits créant une sensation de « fraîcheur » sur la peau (exemple du menthol et des dérivés).

L'invention a aussi pour objet une composition (C1) telles que définie précédemment, pour son utilisation dans une méthode de traitement thérapeutique de l'hypoxie des cellules endothéliales du corps humain ou animal.

L'étude expérimentale suivante illustre l'invention sans toutefois la limiter.

### Exemples de préparation de compositions (C1) selon l'invention

### Exemple 1 : préparation d'une composition A1 comprenant les composés de formules (Ia21) et (Ib21).

On introduit 239 grammes de valine soit un équivalent molaire dans un mélange de 2161grammes constitué par 1512 grammes d'eau et de 649 grammes d'isopropanol compris dans un réacteur en verre à double enveloppe, dans laquelle circule un fluide caloporteur, muni d'une agitation efficace et d'un dispositif de barbotage d'azote par fond de réacteur, à une température de 20°C. Le pH du milieu ainsi préparé est ajusté à une valeur de pH de 10 par ajout d'une solution de soude à 30%. 450 grammes de chlorure d'hexadécanoyle soit 0,8 équivalents molaires sont ensuite ajoutés progressivement sur le milieu à une température comprise entre 20°C et 30°C, de façon à maitriser l'exothermie ; une solution de soude à 30% est parallèlement ajoutée au milieu de façon à maintenir la valeur du pH du milieu à une valeur comprise entre 10 et 10,5.

A la fin de l'addition du chlorure d'hexadécanoyle, le milieu réactionnel est maintenu sous agitation pendant une durée de 2 heures. Le milieu réactionnel est ensuite porté à une température de 55°C sous agitation et une quantité de 469 grammes d'une solution acide d'acide phosphorique à 75% est ensuite progressivement introduite de façon à obtenir une valeur de pH du milieu réactionnel de l'ordre de 2,0. L'agitation est arrêtée et la phase aqueuse du milieu décantée est ensuite soutirée. La phase organique restant dans le réacteur est ensuite lavée par une quantité de 2700 grammes d'une solution aqueuse de chlorure de sodium à 10% à une température de 60°C sous agitation. La phase aqueuse est soutirée par le fond du réacteur et la phase de lavage telle que décrite précédemment est répétée une fois supplémentaire. A l'issu du lavage, la phase organique est séchée par distillation sous vide de l'eau résiduelle.

Une quantité de 32 grammes de 1,3 butanediol soit un équivalent molaire est introduite dans le réacteur comprenant 126 grammes du milieu réactionnel séché, agité et porté à une température de 120°C. Lorsque le mélange est dispersé, une quantité de 0,4 grammes d'acide sulfurique à 98% est introduite dans le réacteur, et le mélange résultant est porté à une température de 120°C, sous un vide partiel avec un barbotage d'azote régulier introduit par le fond du réacteur. Le mélange réactionnel est ensuite maintenu pendant une durée de 24 heures sous agitation et à 120°C, puis neutralisé par ajout d'une solution de soude à 30% de façon à obtenir une valeur de pH de 5% du milieu réactionnel comprise entre 3,0 et 6,0. Le milieu réactionnel est ensuite vidangé et les caractéristiques analytiques de la composition A1 mesurées sont les suivantes :
Indice acide (selon NFT 60-204) = 36,5
pH 5% de la composition A1 dans l'eau (selon la méthode NFT 73-206) = 7,1
Indice d'Hydroxyle (selon U.S. Pharmacopeia XXI NF XVI 01/011985) = 135,5
Indice d'ester (Indice de saponification (selon NFT 60-110) - Indice d'acide) = 100,8

### Exemple 2 : préparation d'une composition A2 comprenant les composés de formules (Ia11) et (Ib11).

Le mode opératoire du procédé décrit dans l'exemple 1 est mis en oeuvre pour un équivalent molaire de valine, 0,8 équivalent molaire de chlorure d'hexadécanoyle et un équivalent molaire de 1,3-propanediol pour obtenir la composition A2 dont les caractéristiques analytiques sont les suivantes :
Indice acide (selon NFT 60-204) = 28,3
pH 5% de la composition A2 dans l'eau (selon la méthode NFT 73-206) = 6,7
Indice d'Hydroxyle (selon U.S. Pharmacopeia XXI NF XVI 01/011985) = 80,5
Indice d'ester (Indice de saponification (selon NFT 60-110) - Indice d'acide) = 121,7

### Exemple 3 : préparation d'une composition B1 comprenant les composés de formules (Ia22) et (Ib22).

Le mode opératoire du procédé décrit dans l'exemple 1 est mis en oeuvre pour un équivalent molaire de valine, 0,8 équivalent molaire de chlorure d'octanoyle et un équivalent molaire de 1,3-butanediol pour obtenir la composition B1 dont les caractéristiques analytiques sont les suivantes :
Indice acide (selon NFT 60-204) = 55,7
pH 5% de la composition B1 dans l'eau (selon la méthode NFT 73-206) = 4,2
Indice d'Hydroxyle (selon U.S. Pharmacopeia XXI NF XVI 01/011985) = 194,6
Indice d'ester (Indice de saponification (selon NFT 60-110) - Indice d'acide) = 128,2

### Exemple 4 : préparation d'une composition B2 comprenant les composés de formules (Ia12) et (Ib12).

Le mode opératoire du procédé décrit dans l'exemple 1 est mis en oeuvre pour un équivalent molaire de valine, 0,8 équivalent molaire de chlorure d'octanoyle et un équivalent molaire de 1,3-propanediol pour obtenir la composition B2 dont les caractéristiques analytiques sont les suivantes :
Indice acide (selon NFT 60-204) = 54,2
pH 5% de la composition B2 dans l'eau (selon la méthode NFT 73-206) = 4,5
Indice d'Hydroxyle (selon U.S. Pharmacopeia XXI NF XVI 01/011985) = 21,2
Indice d'ester (Indice de saponification (selon NFT 60-110) - Indice d'acide) = 159,0

### Exemple 5 : préparation d'une composition D1 comprenant les composés de formules (Ia23) et (Ib23).

Le mode opératoire du procédé décrit dans l'exemple 1 est mis en oeuvre pour un équivalent molaire de valine, 0,8 équivalent molaire de chlorure d'undécylènoyle et un équivalent molaire de 1,3-butanediol pour obtenir la composition D1 dont les caractéristiques analytiques sont les suivantes :
Indice acide (selon NFT 60-204) = 50,0
pH 5% de la composition D1 dans l'eau (selon la méthode NFT 73-206) = 5,3
Indice d'Hydroxyle (selon U.S. Pharmacopeia XXI NF XVI 01/011985) = 149,9
Indice d'ester (Indice de saponification (selon NFT 60-110) - Indice d'acide) = 114,0

### Exemple 6 : préparation d'une composition D2 comprenant les composés de formules (Ia13) et (Ib13).

Le mode opératoire du procédé décrit dans l'exemple 1 est mis en oeuvre pour un équivalent molaire de valine, 0,8 équivalent molaire de chlorure d'undécylènoyle et un équivalent molaire de 1,3-propanediol pour obtenir la composition D2 dont les caractéristiques analytiques sont les suivantes :
Indice acide (selon NFT 60-204) = 38,6
pH 5% de la composition D2 dans l'eau (selon la méthode NFT 73-206) = 5,7
Indice d'Hydroxyle (selon U.S. Pharmacopeia XXI NF XVI 01/011985) = 189,7
Indice d'ester (Indice de saponification (selon NFT 60-110) - Indice d'acide) = 135,1

### Exemple 7 : préparation d'une composition E1 comprenant les composés de formules (Ia24) et (Ib24).

Le mode opératoire du procédé décrit dans l'exemple 1 est mis en oeuvre pour un équivalent molaire de proline, 0,8 équivalent molaire de chlorure d'undécylènoyle et un équivalent molaire de 1,3-butanediol pour obtenir la composition E1 dont les caractéristiques analytiques sont les suivantes :
Indice acide (selon NFT 60-204) = 41,9
pH 5% de la composition E1 dans l'eau (selon la méthode NFT 73-206) = 5,0
Indice d'Hydroxyle (selon U.S. Pharmacopeia XXI NF XVI 01/011985) = 142,6
Indice d'ester (Indice de saponification (selon NFT 60-110) - Indice d'acide) = 116,0

### Exemple 8 : préparation d'une composition E2 comprenant les composés de formules (Ia14) et (Ib14).

Le mode opératoire du procédé décrit dans l'exemple 1 est mis en oeuvre pour un équivalent molaire de proline, 0,8 équivalent molaire de chlorure d'undécylènoyle et un équivalent molaire de 1,3-propanediol pour obtenir la composition E2 dont les caractéristiques analytiques sont les suivantes :
Indice acide (selon NFT 60-204) = 25,6
pH 5% de la composition E2 dans l'eau (selon la méthode NFT 73-206) = 5,3
Indice d'Hydroxyle (selon U.S. Pharmacopeia XXI NF XVI 01/011985) = 149,7
Indice d'ester (Indice de saponification (selon NFT 60-110) - Indice d'acide) = 152,2

### Exemple 9 : préparation d'une composition F1 comprenant les composés de formules (Ia25) et (Ib25).

Le mode opératoire du procédé décrit dans l'exemple 1 est mis en oeuvre pour un équivalent molaire de leucine, 0,8 équivalent molaire de chlorure d'octanoyle et un équivalent molaire de 1,3-butanediol pour obtenir la composition F1 dont les caractéristiques analytiques sont les suivantes :
Indice acide (selon NFT 60-204) = 27,1
pH 5% de la composition F1 dans l'eau (selon la méthode NFT 73-206) = 5,3
Indice d'Hydroxyle (selon U.S. Pharmacopeia XXI NF XVI 01/011985) = 99,9
Indice d'ester (Indice de saponification (selon NFT 60-110) - Indice d'acide) = 169,4

### Evaluation de l'effet de composés et de compositions selon l'invention sur la production d'ATP intracellulaire dans des cultures de cellules endothéliales avant subi un stress oxydant.

### Protocole

Des cellules HUVEC (Human Umbilical Vein Endothelial Cells en langue anglaise) au passage R3 sont ensemencées à 2000 cellules/puits dans des plaques comprenant 96 puits.

Les cellules sont ensuite cultivées en milieu EGM-2 (Endothelial Growth Médium en langue anglaise), commercialisé par la société Lonza, pendant 7 jours à une température de 37°C sous 5% de CO₂.

Les milieux de culture sont alors remplacés par du milieu EGM-2, contenant les dilutions des composés et des compositions à tester.

Des témoins (T) sont également préparés en remplaçant des milieux de culture par le seul milieu EGM-2.

Les cellules présentes dans le milieu EGM-2 et associées aux dilutions des composés et des compositions à tester, ainsi que les témoins (T) sont ensuite incubés pendant une durée de 24 heures à une température de 37°C.

Suite à cette incubation, les milieux des cellules associées aux dilutions des composés et des compositions à tester et les milieux des témoins (T) sont remplacés par un milieu d'EGM-2 supplémenté en eau oxygénée à une concentration de 0,8 millimoles par litre.

On notera par la suite « témoins (T1) » les milieux des témoins (T) supplémentés en eau oxygénée et « témoins (T) » les milieux des témoins (T) non supplémentés en eau oxygénée.

Les cellules associées aux dilutions des composés et des compositions à tester, les témoins (T) et les témoins (T1) sont ensuite incubés pendant 10 minutes à une température de 37°C, puis rincés avec du PBS (Phosphate Buffer Saline).

La quantité d'ATP intracellulaire et la quantité de protéines produites par les cellules associées aux dilutions des composés et des compositions à tester, les témoins (T) et les témoins (T1) sont évaluées après lyse des tapis moléculaires en présence d'un tampon de lyse.

La quantité d'ATP est quantifiée par la méthode luminométrique, mise en oeuvre par l'intermédiaire d'un lecteur de plaques luminomètre de marque FLUOROSKAN ASCENT FL™ commercialisé par la société LABSYSTEMS, et la quantification des protéines est réalisée à l'aide de la méthode BCA. Cette dernière méthode permet de normaliser les quantités d'ATP dosées et d'évaluer la cytotoxycité de chaque condition expérimentale.

Un seuil de cytotoxicité a été fixé à 80% du groupe témoin.

Les résultats sont exprimés en millimole d'ATP produite par milligramme de protéines produites et l'étude statistique des résultats a été réalisée à l'aide du test T de Student bilatéral à variance inégale.

Les effets évalués sur les cellules associées aux dilutions des composés et des compositions à tester, les témoins (T) et les témoins (T1) ont été réalisés sur deux expériences indépendantes et les résultats présentés correspondent à la moyenne des deux essais.

Pour chaque condition expérimentale, les statistiques les plus restrictives ont été appliquées. Les résultats obtenus sont consignés dans le Tableau 1 suivant :

**Tableau 1 : évaluation de l'effet des compositions A1, A2, B1, B2, D1, D2, E1, E2 et F1,sur la production d'ATP par des cellules HUVEC soumises à l'eau oxygénée.**

| Composition testée | Concentration (% p/v) par rapport à l'extrait sec) | Quantité d'ATP produite (mmoles d'ATP/mg de protéines) |
|---|---|---|
| Témoin (T) | - | 0,045 |
| Témoin (T1) | - | 0,016 |
| Composition A1 | 0,00075 | 0,029 |
| Composition A2 | 0,0005 | 0,020 |
| Composition B1 | 0,0005 | 0,021 |
| Composition B2 | 0,0005 | 0,021 |
| Composition D1 | 0,00005 | 0,020 |
| Composition D2 | 0,00005 | 0,024 |
| Composition E1 | 0,00005 | 0,020 |
| Composition E2 | 0,00005 | 0,020 |
| Composition F1 | 0,0005 | 0,018 |

Le stress oxydant procuré par l'ajout d'eau oxygénée sur les cellules témoins (T) induit une diminution de 64% de l'ATP produite par les cellules endothéliales (cellules témoins (T1)). Ce résultat valide ainsi les conditions du test expérimental mis en oeuvre.

Lorsque les cellules endothéliales sont associées à la composition (A1), la quantité d'ATP produite par lesdites cellules endothéliales est en augmentation de 81,25% par rapport aux cellules témoins (T1) soumises au même stress oxydant.

Lorsque les cellules endothéliales sont associées à la composition (D2), la quantité d'ATP produite par lesdites cellules endothéliales est en augmentation de 50,0% par rapport aux cellules témoins (T1) soumises au même stress oxydant.

Lorsque les cellules endothéliales sont associées aux compositions (B1) et (B2), la quantité d'ATP produite par lesdites cellules endothéliales est en augmentation de 31,25% par rapport aux cellules témoins (T1) soumises au même stress oxydant.

Lorsque les cellules endothéliales sont associées aux compositions (A2), (D1), (E1) et (E2), la quantité d'ATP produite par lesdites cellules endothéliales est en augmentation de 25,0 % par rapport aux cellules témoins (T1) soumises au même stress oxydant.

Lorsque les cellules endothéliales sont associées à la composition (F1), la quantité d'ATP produite par lesdites cellules endothéliales est en augmentation de 12,5% par rapport aux cellules témoins (T1) soumises au même stress oxydant.

Aucune des compositions testées n'a induit de cytotoxicité significative selon le test BCA mis en oeuvre avec le kit « BC-assay » commercialisé par la société Interchim.

Il en résulte que les compositions selon l'invention comprenant les composés selon l'invention permettent de ralentir la baisse de la production d'ATP par les cellules endothéliales soumises à des stress oxydants.

### Références bibliographiques citées dans la description

(1) Chang et al.: "Aging and survival of cutaneous microvasculature", J. Invest Dermatol. 2002 May;118(5):752-8.
(2) Chung et al.: "Differential effects of photoaging vs intrinsic aging on the vascularization of human skin" Arch. Dermatol. 2002 Nov;138(11):1437-42.
(3) Toyoda et al.: "Ultrastructural characterization of microvasculature in photoaging" J. Dermatol Sci. 2001 Aug;27 Suppl 1:S32-41.
(4) Janssens, « effect of venotropic drugs on the respiratory activity of isolated mitochondria and in endothelial cells », in British Journal of Pharmacology (2000) 130, 1513-1524.

## Revendications

1. Composition (C1) comprenant pour 100% de sa masse :
- De 99% massique à 20% massique d'au moins un composé de formule (la)
R'-O-A-O-H (la),
formule (la) dans laquelle R' représente soit un radical monovalent de formule (IIa) : 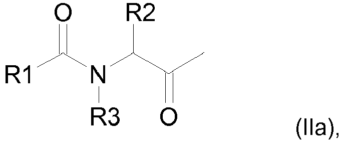 dans laquelle R1 représente un radical aliphatique, saturé ou insaturé, linéaire ou ramifié, comportant de 7 à 30 atomes de carbone, R2 représente un atome d'hydrogène ou un radical choisi parmi les radicaux méthyle, isopropyle, isobutyle, 1-méthyl propyle, hydroxyméthyle, 1-hydroxy éthyle, thiométhyle, 2-méthylthio éthyle, 4-aminobutyle, 3-guanidino propyle, 3-uréido propyle, (1-amino carbonyl) méthyle, 2-(amino carbonyl) éthyle, benzyle, 4-hydroxy benzyle, 3,4-dihydroxy benzyle, [1H-indol-3-yl] méthyle, (1H-imidazol-4-yl) méthyle, 3-amino propyle et R3 représente un atome d'hydrogène ou un radical méthyle ; soit un radical monovalent de formule (IIb) : 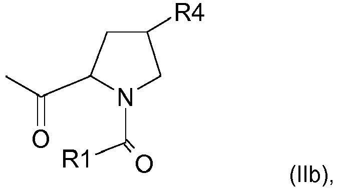 dans laquelle R1 est tel que défini dans la formule (IIa) et R4 représente un atome d'hydrogène ou un radical hydroxy ;
et formule (la) dans laquelle A représente le radical divalent de formule (III) :
-CH(X1)-[C(X2)(X3)]p-CH(X4)- (III)
dans laquelle X1, X2, X3 et X4, identiques ou différents, représentent soit un atome d'hydrogène, soit un radical méthyle, soit un radical éthyle et p représente un nombre entier supérieur ou égal à 1 et inférieur ou égal à 6 ; et
- De 1% massique à 80% massique d'au moins un composé de formule (Ib) :
R-O-A-O-R (Ib),
formule (Ib) dans laquelle R, représente soit un radical monovalent de formule (IIa) : 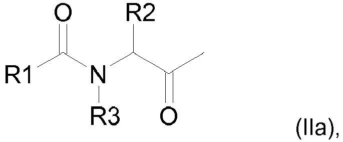 dans laquelle R1 représente un radical aliphatique, saturé ou insaturé, linéaire ou ramifié, comportant de 7 à 30 atomes de carbone, R2 représente un atome d'hydrogène ou un radical choisi parmi les radicaux méthyle, isopropyle, isobutyle, 1-méthyl propyle, hydroxyméthyle, 1-hydroxy éthyle, thiométhyle, 2-méthylthio éthyle, 4-aminobutyle, 3-guanidino propyle, 3-uréido propyle, (1-amino carbonyl) méthyle, 2-(amino carbonyl) éthyle, benzyle, 4-hydroxy benzyle, 3,4-dihydroxy benzyle, [1H-indol-3-yl] méthyle, (1H-imidazol-4-yl) méthyle, 3-amino propyle, et R3 représente un atome d'hydrogène ou un radical méthyle ; soit un radical monovalent de formule (IIb) : 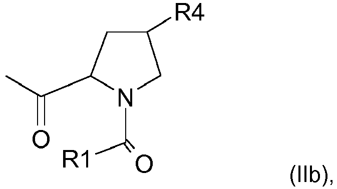 dans laquelle R1 est tel que défini dans la formule (IIa) et R4 représente un atome d'hydrogène ou un radical hydroxy,
et formule (Ib) dans laquelle A représente le radical divalent de formule (III) :
-CH(X1)-[C(X2)(X3)]p-CH(X4)- (III)
dans laquelle X1, X2, X3 et X4, identiques ou différents, représentent soit un atome d'hydrogène, soit un radical méthyle, soit un radical éthyle et P représente un nombre entier supérieur ou égal à 1 et inférieur ou égal à 6.

2. Composition (C1) telle que définie à la revendication 1, pour laquelle dans les formules (IIa) et (IIb), le radical R1-C(=O)- représente un radical choisi parmi les radicaux octanoyle, décanoyle, ω-undécylènoyle, dodécanoyle, tétradécanoyle, hexadécanoyle, octadécanoyle, eicosanoyle, docosanoyle, 9-octadécènoyle, éicosènoyle, 13-docosènoyle, 9,12-octadécadiènoyle ou 9,12, 15-octadécatriénoyle.

3. Composition (C1) telle que définie à l'une ou quelconque des revendications 1 ou 2, pour laquelle dans la formule (IIa), R1 et R3 sont tels que définis précédemment et R2 représente un radical choisi parmi les radicaux, méthyle, isopropyle, isobutyle, 1-méthyl propyle ou benzyle, et dans la formule (IIb), R1 est tel que défini précédemment et R4 représente un atome d'hydrogène.

4. Composition (C1) telle que définie à l'une ou quelconque des revendications 1 à 3, pour laquelle dans les formules (la) et (Ib), A représente le radical divalent de formule (IIIa) :
-(CH₂)_{q}- (IIIa)
dans laquelle q est égal 3, 4 ou à 6..

5. Composition (C1) telle que définie à la revendication 4, pour laquelle dans les formules (la) et (Ib), A représente le radical divalent :
-(CH₂)₃-.

6. Composition (C1) telle que définie à l'une ou quelconque des revendications 1 à 3, pour laquelle dans les formules (la) et (Ib), A représente le radical divalent :
-(CH₂)₂-CH(CH₃)-.

7. Composition (C1) telle que définie à l'une quelconque des revendications 1 à 6, pour son utilisation dans une méthode de traitement thérapeutique de l'hypoxie des cellules endothéliales du corps humain ou animal.

## Patentansprüche

1. Zusammensetzung (C1), umfassend auf 100 % ihrer Masse:
- 99 Masse-% bis 20 Masse-% mindestens einer Verbindung der Formel (la)
R'-O-A-O-H (Ia),
wobei in Formel (la) R' für entweder einen einwertigen Rest der Formel (IIa) steht: 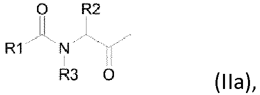 wobei R1 für einen gesättigten oder ungesättigten, geradkettigen oder verzweigten aliphatischen Rest steht, der 7 bis 30 Kohlenstoffatome umfasst, R2 für ein Wasserstoffatom oder einen Rest steht, der aus den Methyl-, Isopropyl-, Isobutyl-, 1-Methylpropyl-, Hydroxymethyl-, 1-Hydroxyethyl-, Thiomethyl-, 2-Methylthioethyl-, 4-Aminobutyl-, 3-Guanidinopropyl-, 3-Ureidopropyl-, (1-Aminocarbonyl)-methyl-, 2-(Aminocarbonyl)-ethyl-, Benzyl-, 4-Hydroxybenzyl-, 3,4-Dihydroxybenzyl-, [1H-Indol-3-yl]-methyl-, (1H-Imidazol-4-yl)-methyl-, 3-Aminopropylresten ausgewählt ist, und R3 für ein Wasserstoffatom oder einen Methylrest steht; oder einen einwertigen Rest der Formel (IIb): 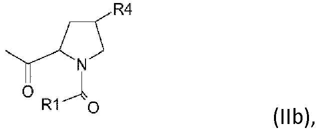 wobei R1 wie in der Formel (IIa) definiert ist und R4 für ein Wasserstoffatom oder einen Hydroxyrest steht;
und wobei in Formel (Ia) A für den zweiwertigen Rest der Formel (III) steht:
-CH(X1)-[C(X2)(X3)]p-CH(X4)- (III),
wobei X1, X2, X3 und X4, identisch oder verschieden, für entweder ein Wasserstoffatom oder einen Methylrest oder einen Ethylrest stehen, und p für eine ganze Zahl von größer oder gleich 1 und kleiner oder gleich 6 steht; und
- 1 Masse-% bis 80 Masse-% mindestens einer Verbindung der Formel (Ib):
R-O-A-O-R (Ib),
wobei in Formel (Ib) R für entweder einen einwertigen Rest der Formel (IIa) steht: 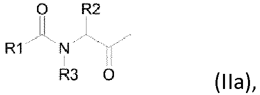 wobei R1 für einen gesättigten oder ungesättigten, geradkettigen oder verzweigten aliphatischen Rest steht, der 7 bis 30 Kohlenstoffatome umfasst, R2 für ein Wasserstoffatom oder einen Rest steht, der aus den Methyl-, Isopropyl-, Isobutyl-, 1-Methylpropyl-, Hydroxymethyl-, 1-Hydroxyethyl-, Thiomethyl-, 2-Methylthioethyl-, 4-Aminobutyl-, 3-Guanidinopropyl-, 3-Ureidopropyl-, (I-Aminocarbonyl)-methyl-, 2-(Aminocarbonyl)-ethyl-, Benzyl-, 4-Hydroxybenzyl-, 3,4-Dihydroxybenzyl-, [1H-Indol-3-yl]-methyl-, (1H-Imidazol-4-yl)-methyl-, 3-Aminopropylresten ausgewählt ist, und R3 für ein Wasserstoffatom oder einen Methylrest steht; oder einen einwertigen Rest der Formel (IIb): 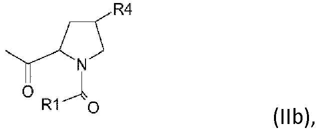 wobei R1 wie in der Formel (IIa) definiert ist und R4 für ein Wasserstoffatom oder einen Hydroxyrest steht,
und wobei in Formel (Ib) A für den zweiwertigen Rest der Formel (III) steht:
-CH(X1)-[C(X2)(X3)]p-CH(X4)- (III),
wobei X1, X2, X3 und X4, identisch oder verschieden, für entweder ein Wasserstoffatom oder einen Methylrest oder einen Ethylrest stehen, und P für eine ganze Zahl von größer oder gleich 1 und kleiner oder gleich 6 steht.

2. Zusammensetzung (C1) wie in Anspruch 1 definiert, wobei in den Formeln (IIa) und (IIb) der Rest R1-C-(=O)- für einen Rest steht, der aus den Octanoyl-, Decanoyl-, ω-Undecylenoyl-, Dodecanoyl-, Tetradecanoyl-, Hexadecanoyl-, Octadecanoyl-, Eicosanoyl-, Docosanoyl-, 9-Octadecenoyl-, Eicosenoyl-, 13-Docosenoyl-, 9,12-Octadecadienoyl- oder 9,12,15-Octadecatrienoylresten ausgewählt ist.

3. Zusammensetzung (C1) wie in einem der Ansprüche 1 oder 2 definiert, wobei in der Formel (IIa) R1 und R3 wie vorstehend definiert sind und R2 für einen Rest steht, der aus den Methyl-, Isopropyl-, Isobutyl-, 1-Methylpropyl- oder Benzylresten ausgewählt ist, und in der Formel (IIb) R1 wie vorstehend definiert ist und R4 für ein Wasserstoffatom steht.

4. Zusammensetzung (C1) wie in einem der Ansprüche 1 bis 3 definiert, wobei in den Formeln (la) und (Ib) A für den zweiwertigen Rest der Formel (IIIa) steht:
-(CH₂)_{q}- (IIIa),
wobei q gleich 3, 4 oder 6 ist.

5. Zusammensetzung (C1) wie in Anspruch 4 definiert, wobei in den Formeln (la) und (Ib) A für den zweiwertigen Rest steht:
-(CH₂)₃-.

6. Zusammensetzung (C1) wie in einem der Ansprüche 1 bis 3 definiert, wobei in den Formeln (la) und (Ib) A für den zweiwertigen Rest steht:
-(CH₂)₂-CH(CH₃)-.

7. Zusammensetzung (C1) wie in einem der Ansprüche 1 bis 6 definiert, zu ihrer Verwendung in einem Verfahren zur therapeutischen Behandlung der Hypoxie der Endothelzellen des menschlichen oder tierischen Körpers.

## Claims

1. Composition (C1) comprising for 100% of its weight:
- From 99% by weight to 20% by weight of at least one compound of formula (Ia)
R'-O-A-O-H (Ia),
formula (Ia) wherein R' is either a monovalent radical of formula (IIa): 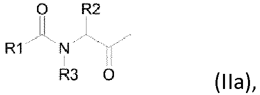 wherein R1 is a saturated or unsaturated, linear or branched alphatic radical, comprising from 7 to 30 carbon atoms, R2 is a hydrogen atom or a radical chosen from the radicals methyl, isopropyl, isobutyl, 1-methyl propyl, hydroxymethyl, 1-hydroxy ethyl, thiomethyl, 2-methylthio ethyl, 4-aminobutyl, 3-guanidino propyl, 3-ureido propyl, (1-amino carbonyl) methyl, 2-(amino carbonyl) ethyl, benzyl, 4-hydroxy benzyl, 3,4-dihydroxy benzyl, [1H-indol-3-yl] methyl, (1H-imidazol-4-yl) methyl, 3-amino propyl and R3 is a hydrogen atom or a methyl radical; or a monovalent radical of formula (IIb): 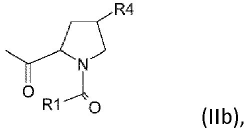 wherein R1 is such as defined in the formula (IIa) and R4 is a hydrogen atom or a hydroxy radical;
and formula (Ia) wherein A is the divalent radical of formula (III):
-CH(X1)-[C(X2)(X3)]p-CH(X4)- (III)
wherein X1, X2, X3 and X4, are identical or different, and are either a hydrogen atom or a methyl radical or an ethyl radical, and p is an integer greater than or equal to 1 and less than or equal to 6; and
- From 1 % by weight to 80% by weight of at least one compound of formula (Ib):
R-O-A-O-R (Ib),
formula (Ib) wherein R, is either a monovalent radical of formula (IIa): 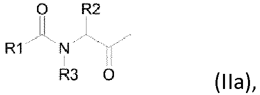 wherein R1 is a saturated or unsaturated, linear or branched alphatic radical, comprising from 7 to 30 carbon atoms, R2 is a hydrogen atom or a radical chosen from the radicals methyl, isopropyl, isobutyl, 1-methyl propyl, hydroxymethyl, 1-hydroxy ethyl, thiomethyl, 2-methylthio ethyl, 4-aminobutyl, 3-guanidino propyl, 3-ureido propyl, (1-amino carbonyl) methyl, 2-(amino carbonyl) ethyl, benzyl, 4-hydroxy benzyl, 3,4-dihydroxy benzyl, [1-indol-3-yl] methyl, (1-imidazol-4-yl) methyl, 3-amino propyl, and R3 is a hydrogen atom or a methyl radical; or a monovalent radical of formula (IIb): 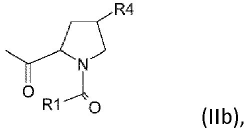 wherein R1 is such as defined in the formula (IIa) and R4 is a hydrogen atom or a hydroxy radical,
and formula (Ib) wherein A is the divalent radical of formula (III):
-CH(X1)-[C(X2)(X3)]p-CH(X4)- (III)
wherein X1, X2, X3 and X4, identical or different, are either a hydrogen atom, or a methyl radical, or an ethyl radical and P is an integer greater than or equal to 1 and less than or equal to 6.

2. Composition (C1) such as defined in claim 1, for which in the formulas (IIa) and (IIb), the radical R1-C(=O)- is a radical chosen from the radicals octanoyl, decanoyl, ω-undecylenoyl, dodecanoyl, tetradecanoyl, hexadecanoyl, octadecanoyl, eicosanoyl, docosanoyl, 9-octadecenoyl, eicosenoyl, 13-docosenoyl, 9,12-octadecadienoyl or 9,12, 15-octadecatrienoyl.

3. Composition (C1) such as defined in one or any of claims 1 or 2, for which in the formula (IIa), R1 and R3 are such as defined hereinabove and R2 is a radical chosen from the radicals methyl, isopropyl, isobutyl, 1-methyl propyl or benzyl, and in the formula (IIb), R1 is such as defined hereinabove and R4 is a hydrogen atom.

4. Composition (C1) such as defined in one or any of claims 1 to 3, for which in the formulas (Ia) and (Ib),A is the divalent radical of formula (IIIa):
-(CH2)_{q}- (IIIa)
wherein q is equal to 3, 4 or to 6.

5. Composition (C1) such as defined in claim 4, for which in the formulas (Ia) and (Ib), A is the divalent radical:
-(CH₂)₃-.

6. Composition (C1) such as defined in one or any of claims 1 to 3, for which in the formulas (Ia) and (Ib),A is the divalent radical:
-(CH₂)₂-CH(CH₃)-.

7. Composition (C1) such as defined in any of claims 1 to 6, for the use thereof in a method for the therapeutic treatment of hypoxia of endothelial cells of the human or animal body.
